# EUROPEAN PATENT SPECIFICATION

(11) **EP 4 307 923 B1**
(45) Date of publication and mention of the grant of the patent: **25.06.2025**
(21) Application number: 22716049.6
(22) Date of filing: 17.03.2022
(51) Int. Cl.: A23L 33/185, A23L 33/19, A23L 33/00

(54) **LIQUID NUTRITIONAL COMPOSITION SUITABLE FOR MUSCLE FUNCTION**
FLÜSSIGE ERNÄHRUNGSZUSAMMENSETZUNG FÜR MUSKELFUNKTION
COMPOSITION NUTRITIONNELLE LIQUIDE ADAPTÉE À LA FONCTION MUSCULAIRE

(30) Priority: 17.03.2021 EP 21163272
(43) Date of publication of application: 24.01.2024
(60) Divisional application: 25177588.8
(73) Proprietor: N.V. Nutricia, 2712 HM Zoetermeer (NL)
(72) Inventor: VAN DIJK-OTTENS, Miriam, 3584 CT Utrecht (NL); DIJK, Francina Jeannette, 3584 CT Utrecht (NL); FURBER, Matthew James Walter, 3584 CT Utrecht (NL)
(74) Representative: Nederlandsch Octrooibureau
(86) International application number: PCT/EP2022/057020
(87) International publication number: WO 2022/195025

(56) References cited:
- EP-B1- 2 424 386
- US-A1- 2013 023 468
- US-A1- 2013 330 424
- US-A1- 2017 196 944
- US-B2- 9 084 804

## Description

### Field of the invention

The invention relates to a liquid nutritional composition comprising a specifically designed protein fraction for use in a human subject suffering from metabolic stress and/or starvation following trauma, inflammation, sepsis, critical illness, stroke, cancer or COPD, and particularly a human elderly subject of at least 50 years of age, for use in prevention and/or treatment of whole body protein metabolism decline or whole body protein function decline, or for use in improving whole body protein metabolism or whole body protein function. The invention is particularly related to prevention and/or treatment of muscle decline, particularly for use in improving muscle function. Given the preferred target population, the invention particularly pertains to tube feeding.

### Background of the invention

US2017/0196944 addresses muscle atrophy or loss of muscle tissue following orthopaedic surgery. On average, patients undergoing for instance a knee or hip replacement, are in the hospital for 1-3 days post-surgery. There is often an additional period of muscle disuse ranging from 7-21 days before the patient can begin physical therapy rehabilitation to restore function in the operative limb. All that time that a load-bearing extremity is not used, it sets in motion a series of metabolic processes that lead to muscle atrophy resulting in loss of muscle strength and muscle mass. In order to increase muscle protein synthesis, US2017/0196944 focuses the administration of leucine, glutamine, arginine and cysteine for stimulating pathways that increase protein synthesis.

There are also many applications searching for ways to stimulate muscle growth in other situations. For instance, reference is made to WO2013/148688, providing nutrition shakes including those especially formulated for promoting muscle growth, those especially formulated to provide balanced nutritional supplements for normal adults, those especially formulated for diabetic adults and those formulated especially for children (e.g., ages 1 to 6). These shakes preferably comprise intact pea and soy protein representing 38 - 52 wt% of total protein, and the amount of milk protein making 48 - 62 wt% of total protein.

Sarcopenia is the syndrome of loss of muscle due to aging. From the age of about 30, humans lose 3 - 8 % of muscle per decade and this accelerates after 60 years of age. Muscle loss can also be the result of inactivity due to bed rest, physical trauma treatment (such as fractures) or impaired mobility. Alternatively, muscle loss can be due to insufficient muscle protein synthesis or to muscle degradation. There is a lot of information available on the prevention of sarcopenia, the stimulation of muscle growth and the stimulation of muscle protein synthesis.

It is, for instance, known that leucine, high-quality proteins such as casein and whey protein, and vitamin D have a positive impact on muscle growth. In fact, it is widely accepted that fast proteins, such as whey protein, are potent stimulators of muscle protein synthesis and whey protein is seen as the gold standard. Reference can for instance be made to EP2590521.

Also situations of metabolic stress and starvation have a negative impact on muscle function.

It is known that loss of muscle protein occurs rapidly in critically ill patients and may result in a loss of up to 18% of muscle mass already in the first 10 days of intensive care unit (ICU) stay, leading to a marked negative total body nitrogen balance. The magnitude of protein loss has been associated with increased morbidity and mortality. Particularly in the case of an elderly subject who suffers from muscle loss due to aging, it remains a challenge to maintain let alone improve muscle function in these situations of critical care. Hence, there is a need for compositions to improve muscle function in subjects suffering from metabolic stress, particularly elderly subjects.

Outside the field of muscle loss, US2013/330424 describes compositions wherein DHA, UMP, B vitamins and antioxidants are identified being the active components for improving activities of daily living (ADL) in persons suffering from early physical exhaustion after exercise, focusing on increasing or maintaining body weight. It exemplifies nutritional compositions incorporating these active components among lists of further ingredients including a specific example with caseinate, soy and pea protein. Protein is not associated with any active role, and in fact kept the same between interventions and control.

WO2020/245299 provides a method of potentiating musculoskeletal effect of one or more anabolic amino acids in an individual in need thereof. The method comprises administering to the individual a composition comprising the one or more anabolic amino acids, the composition further comprising one or more autophagy-inducing amino acids or their precursors in a total amount effective for the composition to be at least neutral regarding autophagy and preferably positive regarding autophagy. US9084804 B2 discloses a method of increasing or maintaining body weight of a human comprising selecting a human with involuntary weight loss and assessing nutritional status of the human and administering to the human a composition including at least two components selected from the group consisting of (i) a nucleoside equivalent, (ii) an n-3 polyunsaturated fatty acid selected from the group consisting of DHA, DPA and EPA, (iii) a vitamin B selected from the group consisting of vitamin B6, vitamin B9 and vitamin B12 (iv) a phospholipid, (v) an antioxidant selected from the group consisting of vitamin C, vitamin E and selenium, and (vi) a choline in a manner to increase or maintain body weight of the human, with the proviso that at least one nucleoside equivalent or at least one vitamin B is present, wherein the composition is administered to the human in a diet without essentially increasing the daily caloric intake of the human.

### Summary of the invention

It is the aim of this invention to provide a liquid nutritional composition for use in prevention and/or treatment of whole body protein metabolism decline or whole body protein function decline, or for use in improving whole body protein metabolism or whole body protein function, in a human subject suffering from metabolic stress and/or starvation following trauma, inflammation, sepsis, critical illness, stroke, cancer or COPD, particularly an elderly human subject of at least 50 years of age, wherein the composition comprises a protein fraction comprising
(a) 15 to 35 weight% of casein ;
(b) 25 to 50 weight% of whey protein ;
(c) 10 to 30 weight% of soy protein ; and
(d) 10 to 30 weight% of pea protein ;
relative to the total protein in the protein fraction, wherein the sum of said proteins preferably equals 100 weight of the protein fraction. The protein fraction preferably comprises
(a) 20 to 30 weight% of casein ;
(b) 30 to 40 weight% of whey protein ;
(c) 15 to 25 weight% of soy protein ; and
(d) 15 to 25 weight% of pea protein ;
relative to the total protein in the protein fraction, wherein the sum of said proteins preferably equals 100 weight of the protein fraction.

Additionally or alternatively, the composition comprises a protein faction comprising a balanced amino acid profile according to the middle column of table 1, preferably table 2 herein.

The inventors found that such a balanced amino acid profile, preferably in the form of a mix of animal and plant-based proteins (casein, whey protein, soy protein and pea protein) was at least as effective in stimulation of whole body protein synthesis in elder mice after starvation than high-quality dairy proteins relatively high in leucine, which animal proteins are typically the golden standard particularly when addressing loss of muscle due to aging. The substantive replacement of casein and whey protein with a mixture of soy and pea protein was found not to reduce protein metabolism.

This is surprising in view of the skilled person's common general knowledge field that good quality animal proteins such as whey protein have a greater ability to enhance muscle protein synthesis rate and support muscle mass than plant-based proteins, based on extensive research as is for instance summarized in the Berrazaga review (Berrazaga et al. "The Role of the Anabolic Properties of Plant- versus Animal-Based Protein Sources in Supporting Muscle Mass Maintenance: A Critical Review" Nutrients (2019) 11, 1825]. The same review also mentions that the postprandial muscle protein synthesis rate upon ingestion of soy protein does not increase to the same extent as whey protein, even in spite of the fact that soy protein is typically regarded as a high-quality vegetable protein. This is based on a number of studies summarized in Table 3 in Berrazaga(2019). It reports that the consumption of soy protein isolate in elderly men in resting conditions did not stimulate the muscle protein synthesis rate, which remained lower than that induced by the consumption of the same amount of whey protein isolate. While there is no report of an effect under stressed conditions, there is no indication in the field that soy protein would have a more positive impact there. Yet, the inventors found that a mix of soy and pea protein could replace these high-quality animal proteins including whey protein without compromising muscle mass protein synthesis rate in especially these circumstances. Without wishing to be tied down to any theory, this surprising effect was believed to be due to the increased demand of specific amino acids in such highly stressed conditions, and the more balanced amino acid profile of the protein blend, indicative of a protein quality that is optimized to those circumstances.

Similar compositions are known from WO2011/093693 to have advantageous effects in terms of reduced stomach coagulation and - associated therewith - improved gastric emptying which is particularly useful in tube feeding hospitalized patients which suffer from upper gastrointestinal complications like reflux, gastrointestinal discomfort, bloating and aspiration pneumonia as a consequence of the administration of caseins and caseinates that coagulate under the pH conditions in the human stomach. There is nothing in WO2011/093710 that would hint the skilled person towards any effect on whole body protein function or whole body protein loss downside in (elderly) patients suffering from (metabolic) stress starvation.

The invention particularly pertains to the use in prevention and/or treatment of muscle decline, particularly for use in improving muscle function.

The invention is not directed to reduced coagulation and increased gastric emptying, and conditions associated with reduced gastric emptying.

### List of figures

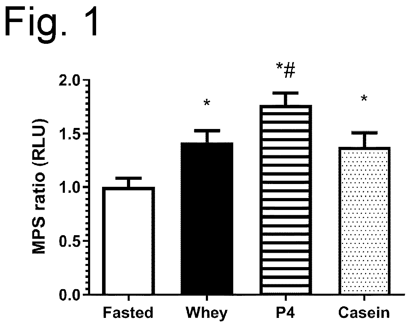

Figure 1 shows the anabolic response to protein supplementation after starvation.

### Detailed description of the invention

The human subject of the invention is suffering from metabolic stress and/or starvation following trauma, inflammation, sepsis, critical illness, stroke, cancer or COPD.

The invention targets a population of human subjects within the group of those who suffer from short-term starvation (< 72 hours) or prolonged starvation (> 72 hours). In the context of the invention, starvation preferably means that the human being has been deprived from food intake for at least 24 hours. Reference is made to Barendregt et al. "Basics in clinical nutrition: simple and stress starvation" e-SPEN - J. Clinical Nutrition and Metabolism (2008) 3, e267-271, its contents herewith incorporated by reference. During simple starvation, humans adapt and use their reserve stores of carbohydrates, fats and protein, and by mechanisms, which reduce energy expenditure as well as conserve protein. Particularly during prolonged starvation, when glycogen levels fall and essential glucose is derived from gluconeogenesis, there appears a negative nitrogen balance and loss of up to 75 g of protein daily.

The expression "metabolic stress" should be understood as a situation during which an unforeseen physical, chemical or biological factor (insult) brutally modifies homeostasis, therefore nutrient's metabolism and nutritional needs of an individual (Colomb, V., Nutrition Clinique et Metabolisme, 2005: 19: 229-33). Metabolic stress (associated with starvation) occurs when he individual is not only starved but subject to the metabolic response to trauma, inflammation, sepsis, critical illness, stroke, cancer or COPD. In these situations, the normal adaptive responses of simple starvation, which conserve body protein, are over-ridden by the neuroendocrine and cytokine effects of injury. Metabolic rate rises rather than falls, ketosis is minimal, protein catabolism accelerates to meet the demands for tissue repair and of gluconeogenesis and there is hyperglycaemia and glucose intolerance.

According to the invention, the human subject is a person suffering metabolic stress and/or starvation following trauma, inflammation, sepsis, critical illness, stroke, cancer or COPD. The subject is not only starved but subject to the metabolic response to trauma, inflammation, sepsis, critical illness, stroke, cancer or COPD. The composition may be provided to such a subject pre- or post-operatively, preferably within 12 hours before and/or after surgery.

Within the context of starvation, the subject suffering from metabolic stress and/or starvation according to the invention is preferably an elderly human. In this respect, it is submitted that in the context of this application, an elderly human is a person of the age of 50 years or more, in particular of the age of 55 or more, more in particular of the age of 60 or more, more in particular of the age of 65 or more. This definition takes into account the fact that the average age varies between different populations, on different continents, etc. Most developed world countries have accepted the chronological age of 65 years as a definition of 'elderly' or older person (associated with the age at which one may begin to receive pension benefits), but like many westernized concepts, this does not adapt well to e.g. the situation in Africa. At the moment, there is no United Nations (UN) standard numerical criterion, but the UN agreed cutoff is 60+ years to refer to the older population in Western world. The more traditional African definitions of an elder or 'elderly' person correlate with the chronological ages of 50 to 65 years, depending on the setting, the region and the country.

In a further preferred embodiment said human preferably suffers from or is at (increased) likelihood of developing muscle decline such as sarcopenia or frailty. Frailty is a condition referring to a collection of symptoms or markers primarily due to the aging-related loss and dysfunction of skeletal muscle, such as: reduced physical activity, muscle weakness, decreased performance, physical weakness, poor endurance, exhaustion, slow walking speed, low muscle strength. In elderly, frailty will increase the risk of adverse events such as death, disability, and institutionalization. Disability refers to the inability to perform a physical activity. The subject preferably suffers from sarcopenia, loss of muscle mass related to aging.

The nutritional composition according to the invention can advantageously be used for prevention and/or treatment of muscle decline, particularly for improving muscle function improving muscle function, particularly increasing muscle protein synthesis. Muscle decline involves insufficient muscle protein synthesis. In the targeted population where muscle function is compromised, promoting muscle protein synthesis is a therapeutic use.

Preferably, the composition of the invention is for therapeutic use in improving whole body protein synthesis, preferably for use in improving muscle protein synthesis.

Within the above embodiments, the inventions is particularly directed to prevention and/or treatment of muscle decline, particularly for use in improving muscle function.

### Composition

According to one embodiment, the liquid nutritional composition according to the invention preferably contains between 10 and 30 gram of proteins per serving, more preferably between 15 and 25 grams serving. The serving preferably has a volume between 50 and 150 ml, preferably about 100 ml.

In the context of this application, when referring to a "protein mixture", a "protein fraction", "proteinaceous matter" or a "protein composition" according to the invention, is meant a collection of proteins, proteinaceous matter, peptides and amino acids, free or in any bound form. Hence, the protein fraction of a nutritional composition is the sum of all proteins, proteinaceous matter, peptides and amino acids, free or in any bound form present in the nutritional composition. Furthermore, the wording "protein mixture" refers to a collection of proteins, proteinaceous matter, peptides and amino acids as such, in any form, as well as to a collection of proteins, proteinaceous matter, peptides and amino acids simultaneously present in a matrix, such as an aqueous matrix, such as a liquid nutritional composition. In the latter case, the protein mixture may be referred to as a protein fraction of that matrix.

The proteins including casein, whey protein, soy protein and pea protein may be substantially in intact form or non-hydrolysed. However, the casein, whey, soy and/or pea protein may also be provided in the form of hydrolysates.

According to a preferred embodiment, the proteins including casein, whey protein, soy protein and pea protein are substantially in intact form or non-hydrolysed. In the context of the invention, a "non-hydrolysed" protein is equivalent to an "intact" protein, meaning that the protein has not been subjected to an hydrolysis process. However, minor amounts of hydrolysed proteins may be present in the source of non-hydrolysed proteins, or may be added to the formulation, such as additional amino acids, such as, for example leucine, isoleucine, glutamine, arginine, or dipeptides and the like. In one embodiment of the present invention, the composition may comprise a free amino acid, or a mixture of free amino acids, up to about 1 g/100 ml, preferably up to about 0.5 gram/100 ml. The protein fraction preferably essentially consists of vegetable and dairy proteinaceous matter, in particular proteins, preferably casein, whey protein, soy protein and pea protein. Small amounts of free amino acids may be accounted for, preferably less than about 5%, more preferably less than about 2%, most preferably less than about 1% of the protein fraction. In a particularly preferred embodiment, there are no free amino acids, peptides or hydrolysates in the composition.

In the context of the invention, the term "about" should preferably be interpreted as a deviation of plus or minus 10 % of the given value.

In one embodiment of the present invention, the viscosity of the liquid enteral nutritional composition is lower than 500 mPa.s, measured at 20 °C (i.e. room temperature) at a shear rate of 100 s⁻¹, preferably between 10 and 200 mPa.s, more preferably between 10 and 100 mPa.s, most preferably below 50 mPa.s. The viscosity may suitably be determined using a rotational viscosity meter using a cone/plate geometry. This viscosity is ideal for orally administering the liquid enteral nutritional composition according to the invention in the form of tube feeding.

In one embodiment of the present invention, the density of the composition ranges between 1.00 g/ml and 1.20 g/ml, especially between 1.05 g/ml and 1.15 g/ml.

According to a further embodiment, the liquid nutritional composition according to the invention optionally comprises one or more of a fat fraction, a carbohydrate fraction, a dietary fibre fraction, and micronutrients. Most preferably, the liquid nutritional composition is sterilized or pasteurized. The liquid nutritional composition is provided in a ready to use liquid form and does not require reconstitution or mixing prior to use.

The composition of the invention may be adapted for tube feeding. The nutritional composition according to the invention preferably has the form of a complete food, i.e. it can meet all nutritional needs of the user. As such, the liquid nutritional composition according to the invention preferably contains 1000 to 2500 kcal per daily dosage. Depending on the condition of the patient, a daily dose is about 25 to 35 kcal/kg bodyweight/day. Therefore, a typical daily dose for a 70 kg person contains about 2000 kcal.

The complete food can be in the form of multiple dosage units, e.g. from 8 (250 ml/unit) to 2 units (1 l/unit) per day for an energy supply of 2000 kcal/day using a liquid enteral nutritional composition according to the invention of 1.0 kcal/ml.

Preferably, the nutritional composition is packaged, stored and provided in a tube feeding bag. Tube feeding is given to provide nutrition to patients which cannot obtain nutrition by swallowing, using a device such as a nasogastric feeding tube or a naso jejunal feeding tube, or by using a percutaneous endoscopic gastrostomy (PEG) or PEG-jejuno-feeding system. In the context of this application, the state of being fed by a feeding tube is called enteral feeding, comprising all of the abovementioned tube feeding systems, and the nutrition used in the feeding a feeding tube is called enteral nutrition. The (tube feed) composition preferably provides 1.0 - 1.8 g protein/kg body weight per day, more preferably 1.0 - 1.8 g protein/kg body weight per day.

### Preferred protein fraction

According to a preferred embodiment, the protein fraction according to the invention comprises casein, whey protein, soy protein and pea protein, wherein the composition comprises a protein fraction comprising
(a) 22 to 28 weight% of casein ;
(b) 32 to 38 weight% of whey protein ;
(c) 17 to 23 weight% of soy protein ; and
(d) 17 to 23 weight% of pea protein ;
relative to the total protein in the protein fraction. In a most preferred embodiment, protein fraction essentially consists of casein, whey protein, soy protein and pea protein i.e. (a) - (d). The proteins may be hydrolysed or intact.

Additionally or alternatively, the composition comprises a protein fraction providing a balanced amino acid pattern, with numbers in gram per 100 gram of the protein fraction:

| | |
|---|---|
| Histidine | 2.0 to 2.6 |
| Isoleucine | 5.2 to 6.4 |
| Leucine | 9.0 to 11.0 |
| Lysine | 7.5 to 9.0 |
| Methionine | 1.7 to 2.3 |
| Cysteine | 1.1 to 1.7 |
| Threonine | 4.9 to 6.2 |
| Tryptophan | 1.2 to 1.6 |
| Valine | 5.5 to 6.9 |
| Phenylalanine | 4.2 to 5.2 |
| Tyrosine | 3.7 to 4.7 |

In a preferred embodiment, the protein fraction provides a balanced amino acid pattern according to the middle column of table 2 herein.

In a most preferred embodiment, the sum of said casein, whey protein, soy protein and pea protein equals 100 weight% of the total weight of the protein fraction i.e. the protein fraction consists of casein, whey protein, soy protein and pea protein. Preferably, all proteins are in substantially intact form.

The aforementioned protein fraction has an excellent amino acid profile, which at least meets and preferably exceeds the WHO amino acid profile recommendations for complete nutrition.

In one embodiment, the protein fraction according to the invention has the essential amino acid profile range as given in the middle column of Table 1 in gram per 100 gram of the protein fraction. In the right column, the minimum amount according to WHO 2007 Guidelines is given.

**Table 1: Amino acid profile per 100 g total protein**

| **Amino acid (essential and semi-essential)** | **Range according to invention (g/100 g)** | **Minimum amount (WHO, 2007)^{a} (g/100 g)** |
|---|---|---|
| Histidine | 2.0 to 2.6 | 1.5 |
| Isoleucine | 5.2 to 6.4 | 3.0 |
| Leucine | 9.0 to 11.0 | 5.9 |
| Lysine | 7.5 to 9.0 | 4.5 |
| Methionine | 1.7 to 2.3 | 1.6 |
| Cysteine | 1.1 to 1.7 | 0.6 |
| Threonine | 4.9 to 6.2 | 2.3 |
| Tryptophan | 1.2 to 1.6 | 0.6 |
| Valine | 5.5 to 6.9 | 3.9 |
| Phenylalanine | 4.2 to 5.2 | Phe + Tyr = 3.0 |
| Tyrosine | 3.7 to 4.7 | |

| | | |
|---|---|---|
| a : based on mean nitrogen requirement of 105 mg nitrogen/kg per day (0.66 g protein/kg body weight per day). | | |

In a further embodiment, the protein fraction according to the invention has the amino acid profile range as given in the middle column of Table 2 in gram per 100 gram of the protein fraction, or more preferably the specific amino acid profile as given in the right column of Table 2, still allowing for a deviation of plus or minus 10 % of the given value.

**Table 2: Amino acid profile per 100 g total protein**

| Amino acid (essential, semi-essential and non-essential) | **Range according to invention (g/100 g)** | **Specific amino acid profile (g/100 g)** |
|---|---|---|
| Histidine | 2.0 to 2.6 | 2.3 |
| Isoleucine | 5.2 to 6.4 | 5.8 |
| Leucine | 9.0 to 11.0 | 9.8 |
| Lysine | 7.5 to 9.0 | 8.3 |
| Methionine | 1.7 to 2.3 | 2.0 |
| Cysteine | 1.1 to 1.7 | 1.4 |
| Threonine | 4.9 to 6.2 | 5.6 |
| Tryptophan | 1.2 to 1.6 | 1.4 |
| Valine | 5.5 to 6.9 | 6.2 |
| Phenylalanine | 4.2 to 5.2 | 4.8 |
| Tyrosine | 3.7 to 4.7 | 4.1 |
| Alanine | 4.0 to 5.1 | 4.6 |
| Arginine | 4.5 to 5.7 | 5.2 |
| Sum of Aspartic Acid and Asparagine | 9.5 to 11.7 | 10.7 |
| Sum of Glutamic acid and Glutamine | 18.0 to 22.8 | 20.2 |
| Glycine | 2.6 to 3.2 | 2.9 |
| Proline | 5.8 to 7.3 | 6.5 |
| Serine | 5.3 to 6.5 | 5.9 |

The protein fraction can be prepared for instance using the method as detailed in WO2011/093710.

### Pea Protein

In the past, pea protein alone is generally classed as quite a poor vegetable source of protein, having a Biological Value (BV) of about 49 % when compared to e.g. whole egg (100 %), cow's milk (91 %), casein (77 %), soy (74 %) and wheat (54 %)(see e.g. Renner, E. (1983) Milk and dairy products in human nutrition. Volkswirtschaftlicher Verlag, Munich, Germany) and having an amino acid score (AAS) which is below the one for whole egg (1), cow's milk (1), casein (1) and soy (0.91). The BV of a protein is the amount of nitrogen used for tissue formation divided by the amount absorbed from the food and is expressed as a percentage. The AAS is the ratio between the amount of the first limiting amino acid in the protein under study (mg/g) and the amount of that amino acid in a reference protein (mg/g), optionally multiplied by the true digestibility (Protein Digestibility Corrected-AAS, PDCAA). According to the WHO (2007) recommendations on protein quality as the reference, pea has an amino acid score of below 1.0 due to the relatively low methionine content. Further, pea protein tastes quite bad (even in intact form) and it doesn't mix too well, leaving a kind of grainy texture to the protein.

Yet, in spite of the above, it was found that pea protein contributes to forming a good overall mix of amino acids. While whey proteins enter the blood stream very fast, pea proteins are absorbed much slower. Pea protein is quite high in cysteine content and can therefore compensate the inadequate amount of cysteine in casein proteins. Furthermore, pea protein is quite high in arginine compared to casein, soy or whey protein which is required for muscle metabolism and which facilitates the intake of body mass while reducing body fat; and it is quite high in lysine, which is needed to build protein muscle and assist in the maintenance of lean body mass.

Several pea sources are readily available to the skilled person, for example, from Roquette (Lestrem, France) which markets a pea isolate obtained from the yellow pea (*Pisum sativum*), and from Cosucra Groupe Warcoing (Warcoing, Belgium).

### Soy protein

Soy protein is categorized as a high-quality, complete protein although the methionine level is slightly below the WHO 2007 recommendation for methionine content. Soy proteins can be divided into different categories according to their production method. Soy protein isolate (SPI) is the most refined form of soy protein. Soy protein isolate contains about 90 percent protein. Soy protein concentrate (SPC) is basically soybean without the water soluble carbohydrates. It contains about 70 percent of protein. Several soy sources are readily available to the skilled person, for example, from The Solae Company (St. Louis, MO, USA).

### Whey Proteins

One of the most superior classes of food protein is whey protein. It is known for its excellent amino acid profile, and for its ability to increase the protein synthesis in a mammal (due to a higher leucine content). Nutritionally speaking, whey protein is known as a naturally complete protein because it contains all of the essential amino acids required in the daily diet. It is also one of the richest sources of branched chain amino acids (BCAAs, in particular leucine) which play an important role in muscle protein synthesis. Whey protein is the preferred choice of proteins to treat persons suffering from sarcopenia. However, as shown in the experimental parts, whey protein in itself can only stimulate muscle protein synthesis in the context of a subject suffering from starvation to a limited extent.

As a source of whey protein to be used in the present invention, any commercially available whey protein source may be used, i.e. whey obtained by any process for the preparation of whey known in the art, as well as whey protein fractions prepared thereof, such as liquid whey, or whey in powder form, such as whey protein isolate (WPI) or whey protein concentrate (WPC). Whey protein concentrate is rich in whey proteins, but also contains other components such as fat, lactose and glycomacroprotein (GMP), a casein-related non-globular protein. Typically, whey protein concentrate is produced by membrane filtration. On the other hand, whey protein isolate consists primarily of whey proteins with minimal amounts of fat and lactose. Whey protein isolate usually requires a more rigorous separation process such as a combination of microfiltration and ultra-filtration or ion exchange chromatography. It is generally understood that a whey protein isolate refers to a mixture in which at least 90 weight% of the solids are whey proteins. A whey protein concentrate is understood as having a percentage of whey proteins between the initial amount in the by-product (about 12 weight%) and a whey protein isolate. In particular, sweet whey, obtained as a by-product in the manufacturing of cheese, acid whey, obtained as a by-product in the manufacturing of acid casein, native whey, obtained by milk microfiltration or rennet whey, obtained as a by-product in the manufacturing of rennet casein, may be used alone or in combination as source of globular whey proteins. Furthermore, whey proteins may originate from all kinds of mammalian animal species, such as, for instance cows, sheep, goats, horses, buffalo's, and camels. Preferably, the whey protein is of bovine origin. Preferably, the whey protein source is available as a powder, preferably the whey protein source is a WPC or WPI.

### Casein/Caseinate

Casein tends to form a gel in the stomach, which slows the digestion. Like many other nutritional compounds, casein is typically bound to a metal ion since the molecule is more stable this way. Specifically, casein is most commonly bound to calcium (Ca²⁺) and sodium (Na⁺) since all of these ions are found naturally in milk, or even potassium (K⁺) or magnesium (Mg²⁺), and tend to stick to the casein during the extraction process. Nutritionally, these compounds are basically interchangeable in the context of the invention, as all forms of casein are effective protein sources. Micellar casein refers to casein in the form of native micelles. It is a high quality milk protein and naturally occurring in milk in a concentration of about 2.6 g/100 ml (Dairy Science and Technology, Walstra et al., CRC Press, 2006). It is concentrated by a process that does not, or does not substantially denature the casein proteins and it is marketed as Micellar Casein Isolate (MCI). Fresh skim milk is subjected to a microfiltration process, in much the same process used to concentrate whey protein, to produce a pure, substantially undenaturated milk protein with its native structure. The resulting material contains between 90 % and 95 %, preferably more than 95 % by weight of micellar casein, the rest mainly being whey protein and other non-protein nitrogen and other constituents, such as lactose and inorganic salts, in particular calcium phosphate.

Within the context of this invention, it is understood that micellar casein may also be provided by other milk protein sources, such as, for instance, sources with essentially preserve the natural 80:20 ratio of casein to whey, such as Milk Protein Concentrate (MPC), which is a powder product usually prepared by ultrafiltration with an average protein content of about 80 weight%, Milk Protein Isolate (MPI), a powder product usually prepared by precipitation with an average protein content of more than 85 weight%, and skimmed concentrated milk.

Within the context of this invention, with the term "casein" both caseinate and micellar casein is indicated. In one embodiment, the casein is caseinate, preferably Na- caseinate or Ca-caseinate. Preferably, the caseinate is Ca-caseinate.

The invention will now be further elucidated by the example here below, without being limited thereby.

### Example

### Introduction

It is well established that animal proteins quality can affect subsequent muscle protein synthesis (MPS). The aim of the study was to investigate the effect of a protein blend from different plant-based and animal protein sources (soy, pea, whey and casein), compared to single animal protein sources on MPS in aged mice undergoing starvation. The model uses a food deprivation time of longer than 24 hours which is described as starvation. Metabolic stress is induced by starvation.

It is known from literature that metabolically stressed (average starvation period over 16 hours (Jensen, 2013)), 25 months of aged mice show 60% of sarcopenic features like loss of body weight, muscle mass, lowered activity, decreased muscle function and declined anti-oxidant levels in plasma.

### Animals

Aged male C57/BL6J mice of 25 months of age were obtained from Janvier Labs (Saint Berthevin, France). Animals were individually housed in a climate-controlled room (12:12 dark/light cycle (lights on: 7 a.m.) with a constant room temperature of 21±1°C). Housing consisted of Makrolon Type III cages (Tecniplast, Italy) with bedding and tissues. Mice were fed ad libitum with a standard diet (AIN93M, Sniff, Germany) and had free access to tap water. All experimental procedures were approved by an Animal Ethical Committee (DEC consult, Soest, the Netherlands) and complied with the principles. of good laboratory animal care following the European Directive for the protection of animal used for scientific purposes. The animals were cared for according to the NIH Guide for the Care and Use of Laboratory Animals. Upon arrival, the mice were allowed to acclimatize for 2 weeks and were fasted overnight (24 hours) before section. At the day of section, mice were randomized to 3 groups (fasted, whey, P4) with no greater deviation than 5% from the overall mean bodyweight (n=8 per group).

### In vivo Muscle protein synthesis (MPS)

MPS was measured with the SUnSET method as previously described by Goodman *et al..* The SUnSET method is an alternative method to determine MPS compared to radioactive isotope or stable isotope tracers. Goodman et al. validated the SUnSET method with a 3H-phenylalanine flooding method in ex vivo plantaris muscle from mice that had received synergist ablation (SA). The results showed that the SUnSET technique was indistinguishable from a standard radioactive based or a standard stable isotope incorporation technique in detecting SA-induced increases in protein synthesis. It had previously been established that when protein synthesis was either measured in myotubes after a 50 min incubation with L-1-13C]valine by measuring tracer enrichment, or after a 30 min incubation with puromycin by quantifying incorporation of puromycin into peptides, results were comparable. This technique was successfully applied in a previous study were similar dosages of protein showed an anabolic response in adult mice. This proves the reliability of the SUnSET method. This method also made it possible to determine protein synthesis rate.

At section day, mice received an oral gavage (end volume 0.5 mL) containing either raw material of 70 mg of:
- Whey protein (source: Lacprodan-9114), positive control (n=8)
- P4, a mixture of 28,59 mg whey, 19,01 mg casein, 15,86 mg pea and 14,66 mg soy (35% whey protein, 25% casein, 20% soy protein and 20% pea protein; n=9)
- casein (n=10).

The amount of leucine was 7.0, 5.3 and 6.1 mg per 0.5 ml water, respectively. No free leucine was added. The postprandial conditions were compared with the fasting state (0.5 mL tap water) to indicate the levels of postprandial increase. 30 min after oral gavage adult mice received a s.c. injection with 0.04 mmol/g bodyweight puromycin (Calbiochem). After an additional 30 min, mice were euthanized by cardiac puncture under total isoflurane anaesthesia (isoflurane/N₂O/O₂). As a result, MPS was measured after a 60 min postprandial period. Plasma and serum samples were prepared by centrifugation and hind limb muscles were excised, weighted, frozen in liquid nitrogen and stored at -80°C until analysis.

The left *tibialis anterior* (TA) muscle was used to determine MPS, expressed as an 'MPS ratio' with fasting values as reference (negative control). Contents of stomachs were scraped and protein content was examined.

### Western blot analysis

Left *tibialis anterior* muscles were cut into pieces and WB buffer (40 mM Tris pH 7.5; 1 mM EDTA; 5 mM EGTA; 10% glycerol; 1% Triton X-100; PhosSTOP Phosphatase Inhibitor Cocktail (Roche Diagnostics) and Protease Inhibitor Ultra Tablets (Roche Diagnostics), 1 tablet per 10 mL buffer) was added in a ratio of 10 ml/mg muscle. Muscles were homogenized using a FastPrep-24 (MP Biomedicals) with 3 chrome-steel beads per tube and run 3 times for 30 s on 6.5 m/s speed. Between rounds, tubes were placed on ice to prevent heating of the samples. Subsequently, samples were continuously shaken at 4°C for 1 h. Protein content was determined using the BCA protein assay kit (Pierce). Muscle homogenates were dissolved in Laemmli buffer (BioRad) and 20e35 mg was loaded on a 4e15% gradient gel (Criterion TGX Precast Gels, BioRad). For each experiment, a pool of the homogenates of mice in fasting state was made and loaded in threefold on each gel to be able to compare samples from different gels. Electrophoresis was performed at 100 V. Proteins were transferred to a 0.2 mm PVDF membrane (BioRad). Membranes were blocked with 5% Protifar protein powder (Nutricia) in TBST buffer (25 mM Tris, pH 7.6; 150 mM NaCl; 0.1% Tween-20) for 1 h followed by overnight incubation at 4°C with anti-puromycin antibody (clone 12D10, 1:5000, Merck Millipore) in 1% BSA in TBST. Membranes were washed 4 times in TBST and incubated for 1 h at room temperature with HRP-conjugated anti-mouse IgG-Fc2a antibody (1:50.000, Jackson Immuno Research) for anti-puromycin antibody. After 4 times washing with TBST, membranes were incubated for 5 min with ECL substrate (SuperSignal West FEMTO, Pierce). Protein synthesis was determined by the density of the whole lane using a Chemidoc XRS (BioRad) and calculated as a relative ratio to the fasted control group. mTOR pathway proteins were determined by the density of the specific band representing the phosphorylated or total protein as indicated by the supplier. After analysis, blots were stained with Coomassie Brilliant Blue R- 250 (BioRad) for 30 min and destained in destain solution (10% acetic acid, 40% methanol) to correct for protein loading differences between lanes.

### Statistical analyses

All data are expressed as means±SEM. Statistical analyses were performed using IBM SPSS Statistics (version 19; SPSS Inc, Chicago, IL). Univariate ANOVA followed by LSD post hoc analysis was used to compare groups. Statistical significance is defined as P < 0.05.

### Results

As shown in figure 1, all protein supplements resulted in statistically significant higher MPS compared to fasting (P<0.05). However, P4 resulted in higher MPS compared to other proteins with statistically significant higher MPS compared to whey protein (P=0.042). Stomach protein content was similar in all condition, suggesting all supplements entered the gastro-intestinal tract.

### Discussion

To the inventors' best knowledge, this was the first experiment to investigate a blend of four different animal and plant-based proteins (P4), compared to single animal protein sources, on MPS in aged mice. P4 was at least as effective in stimulating MPS compared to whey in aged mice during starvation. Without wishing to be tied down to any theory, this may be explained by the higher protein quality and increased rate limiting essential and non- and conditionally essential amino acids within the P4 blend (see table 1), which may reduce the amount of transamination occurring under the metabolic stress and/or starvation, thus increasing the amino acid availability for MPS after splanchnic area amino acid extraction. This may well compensate for the less quality compared to the whey protein standard.

### Conclusion

Compared with single animal protein sources, a mix of animal and plant-based proteins was at least as effective in stimulation of muscle protein synthesis in mice after starvation. This was likely due to the increased demand of specific amino acids in highly stressed conditions and the more balanced amino acid profile of the protein blend, indicative of higher protein quality.

Metabolically stressed (average starvation period 16 hours), 25 months old mice have a mean body weight of 35 grams. Following the calculations of Nair(2016), a human equivalent dose (HED) of the protein supplements can be estimated. In this case mice were orally forced fed (gavaged) with about 70 mg of total proteins, acutely and in 1 bolus. Considering a daily consumption of 150 - 250 g protein per day for a person with a body weight of 80 kg, this would amount to 0.8 - 1.3 gram protein per day. Taking the above supplement numbers as single servings, these numbers would compare to an equivalent of 14 grams of protein to a human of 80 kg average weight. However, the above conversion estimates are believed to be suboptimal, and a more optimal translation into human dosages would be of the order of 20 grams of protein per serving, or 1.1 - 1.8 g/kg body weight per day.
1. Goodman, C.A. and T.A. Hornberger, Measuring protein synthesis with SUnSET: a valid alternative to traditional techniques? Exerc Sport Sci Rev, 2013. 41(2): p. 107-15.
2. Goodman, C.A., et al., Novel insights into the regulation of skeletal muscle protein synthesis as revealed by a new nonradioactive in vivo technique. FASEB J, 2011. 25(3): p. 1028-39.
3. Jensen, T.L., et al., Fasting of mice: a review. Lab Anim, 2013. 47(4): p. 225-40.
4. Nair, A.B. and S. Jacob, A simple practice guide for dose conversion between animals and human. J Basic Clin Pharm, 2016. 7(2): p. 27-31.

## Claims

1. A liquid nutritional composition for use in prevention and/or treatment of whole body protein metabolism decline or whole body protein function decline, or for use in improving whole body protein metabolism or whole body protein function, in a human subject suffering from starvation and/or metabolic stress following trauma, inflammation, sepsis, critical illness, stroke, cancer or COPD, wherein the human subject is preferably a human elderly subject of at least 50 years of age,
wherein the composition comprises a protein fraction comprising
(a) 15 to 35 weight% of casein ;
(b) 25 to 50 weight% of whey protein ;
(c) 10 to 30 weight% of soy protein ; and
(d) 10 to 30 weight% of pea protein ;
relative to the total protein in protein fraction, wherein the sum of said proteins preferably equals 100 weight% of the protein fraction.

2. The liquid nutritional composition for use according to claim 1,
wherein the composition comprises a protein fraction comprising
(a) 20 to 30 weight% of casein ;
(b) 30 to 40 weight% of whey protein ;
(c) 15 to 25 weight% of soy protein ; and
(d) 15 to 25 weight% of pea protein ;
relative to the total protein in the protein fraction, wherein the sum of said proteins preferably equals 100 weight% of the protein fraction.

3. The liquid nutritional composition for use according to claim 1 or 2,
wherein the protein fraction provides a balanced amino acid pattern, with numbers in gram per 100 gram of the protein fraction:
| | |
|---|---|
| Histidine | 2.0 to 2.6 |
| Isoleucine | 5.2 to 6.4 |
| Leucine | 9.0 to 11.0 |
| Lysine | 7.5 to 9.0 |
| Methionine | 1.7 to 2.3 |
| Cysteine | 1.1 to 1.7 |
| Threonine | 4.9 to 6.2 |
| Tryptophan | 1.2 to 1.6 |
| Valine | 5.5 to 6.9 |
| Phenylalanine | 4.2 to 5.2 |
| Tyrosine | 3.7 to 4.7 |

4. The liquid nutritional composition for use according to any one of the preceding claims, for use in prevention and/or treatment of muscle decline, or for use in improving muscle function.

5. The liquid nutritional composition for use according to any one of the preceding claims, wherein the proteins are in hydrolysed or intact form, preferably in intact form.

6. The liquid nutritional composition for use according to claim 5, wherein there are no free amino acids, peptides or hydrolysates in the composition.

7. The liquid nutritional composition for use according to any one of the preceding claims, wherein the human subject is a human elderly subject of at least 60 years of age.

8. The liquid nutritional composition for use according to any one of claims 4 - 7, wherein improving muscle function involves improving muscle protein synthesis.

9. The liquid nutritional composition for use according to any one of the preceding claims, wherein the human subject is a human elderly subject who is suffering or at risk of developing sarcopenia or frailty.

10. The liquid nutritional composition for use according to any one of the preceding claims, wherein the daily amount of protein administered to the human subject is between 1.0 and 1.8 g/kg body weight.

11. The liquid nutritional composition for use according to any one of the preceding claims, wherein the composition is adapted for tube feeding.

12. The liquid nutritional composition for use according to any one of claims 1 - 9, wherein the amount of protein administered to the human subject is between 10 and 30 g per serving, preferably 15 - 25 g per serving.

## Patentansprüche

1. Flüssige Nährstoffzusammensetzung zur Verwendung bei der Vorbeugung und/oder Behandlung einer Verschlechterung des Proteinstoffwechsels oder der Proteinfunktion des gesamten Körpers oder zur Verwendung bei der Verbesserung des Proteinstoffwechsels oder der Proteinfunktion des gesamten Körpers bei einem Menschen, der an Hunger und/oder Stoffwechselstress in der Folge eines Traumas, einer Entzündung, einer Sepsis, einer kritischen Erkrankung, eines Schlaganfalls, Krebs oder COPD leidet, wobei der Mensch bevorzugt ein älterer Mensch von mindestens 50 Jahren ist,
wobei die Zusammensetzung eine Proteinfraktion umfasst, umfassend
(a) 15 bis 35 Gew.-% Casein;
(b) 25 bis 50 Gew.-% Molkenprotein;
(c) 10 bis 30 Gew.-% Sojaprotein; und
(d) 10 bis 30 Gew.-% Erbsenprotein;
relativ zum Gesamtprotein in der Proteinfraktion, wobei die Summe der Proteine bevorzugt 100 Gew.-% der Proteinfraktion entspricht.

2. Flüssige Nährstoffzusammensetzung zur Verwendung nach Anspruch 1, wobei die Zusammensetzung eine Proteinfraktion umfasst, umfassend
(a) 20 bis 30 Gew.-% Casein;
(b) 30 bis 40 Gew.-% Molkenprotein;
(c) 15 bis 25 Gew.-% Sojaprotein; und
(d) 15 bis 25 Gew.-% Erbsenprotein;
relativ zum Gesamtprotein in der Proteinfraktion, wobei die Summe dieser Proteine bevorzugt 100 Gew.-% der Proteinfraktion entspricht.

3. Flüssige Nährstoffzusammensetzung zur Verwendung nach Anspruch 1 oder 2, wobei die Proteinfraktion ein ausgewogenes Aminosäuremuster bereitstellt, mit den folgenden Zahlen in Gramm pro 100 Gramm der Proteinfraktion:
| | |
|---|---|
| Histidin | 2,0 bis 2,6 |
| Isoleucin | 5,2 bis 6,4 |
| Leucin | 9,0 bis 11,0 |
| Lysin | 7,5 bis 9,0 |
| Methionin | 1,7 bis 2,3 |
| Cystein | 1,1 bis 1,7 |
| Threonin | 4,9 bis 6,2 |
| Tryptophan | 1,2 bis 1,6 |
| Valin | 5,5 bis 6,9 |
| Phenylalanin | 4,2 bis 5,2 |
| Tyrosin | 3,7 bis 4,7 |

4. Flüssige Nährstoffzusammensetzung zur Verwendung nach einem der vorstehenden Ansprüche, zur Verwendung bei der Vorbeugung und/oder Behandlung von Muskelschwund oder zur Verwendung bei der Verbesserung der Muskelfunktion.

5. Flüssige Nährstoffzusammensetzung zur Verwendung nach einem der vorstehenden Ansprüche, wobei die Proteine in hydrolysierter oder intakter Form, bevorzugt in intakter Form, vorliegen.

6. Flüssige Nährstoffzusammensetzung zur Verwendung nach Anspruch 5, wobei darin keine freien Aminosäuren, Peptide oder Hydrolysate sind.

7. Flüssige Nährstoffzusammensetzung zur Verwendung nach einem der vorhergehenden Ansprüche, wobei der Mensch ein älterer Mensch mit einem Alter von mindestens 60 Jahren ist.

8. Flüssige Nährstoffzusammensetzung zur Verwendung nach einem der Ansprüche 4 - 7, wobei die Verbesserung der Muskelfunktion die Verbesserung der Muskelproteinsynthese beinhaltet.

9. Flüssige Nährstoffzusammensetzung zur Verwendung nach einem der vorstehenden Ansprüche, wobei der Mensch ein älterer Mensch ist, der an Sarkopenie oder Gebrechlichkeit leidet oder ein Risiko für deren Entwicklung aufweist.

10. Flüssige Nährstoffzusammensetzung zur Verwendung nach einem der vorstehenden Ansprüche, wobei die tägliche Menge an Protein, die dem Mensch verabreicht wird, zwischen 1,0 und 1,8 g/kg Körpergewicht ist.

11. Flüssige Nährstoffzusammensetzung zur Verwendung nach einem der vorhergehenden Ansprüche, wobei die Zusammensetzung für die Sondenernährung angepasst ist.

12. Flüssige Nährstoffzusammensetzung zur Verwendung nach einem der Ansprüche 1-9, wobei die dem Menschen verabreichte Proteinmenge zwischen 10 und 30 g pro Portion, bevorzugt 15 - 25 g pro Portion, ist.

## Revendications

1. Composition nutritionnelle liquide pour utilisation dans la prévention et/ou le traitement du déclin du métabolisme des protéines du corps entier ou du déclin de la fonction des protéines du corps entier, ou pour utilisation dans l'amélioration du métabolisme des protéines du corps entier ou de la fonction des protéines du corps entier, chez un sujet humain souffrant de famine et/ou de stress métabolique à la suite d'un traumatisme, d'une inflammation, d'une septicémie, d'une maladie grave, d'un accident vasculaire cérébral, d'un cancer ou d'une BPCO, dans laquelle le sujet humain est de préférence un sujet humain âgé d'au moins 50 ans,
dans laquelle la composition comprend une fraction de protéines comprenant
(a) de 15 à 35 % en poids de caséine ;
(b) de 25 à 50 % en poids de protéine de lactosérum ;
(c) de 10 à 30 % en poids de protéine de soja ; et
(d) de 10 à 30 % en poids de protéine de pois ;
par rapport aux protéines totales dans la fraction protéique, dans laquelle la somme desdites protéines est de préférence égale à 100 % en poids de la fraction protéique.

2. Composition nutritionnelle liquide pour utilisation selon la revendication 1,
dans laquelle la composition comprend une fraction protéique comprenant
(a) de 20 à 30 % en poids de caséine ;
(b) de 30 à 40 % en poids de protéine de lactosérum ;
(c) de 15 à 25 % en poids de protéine de soja ; et
(d) de 15 à 25 % en poids de protéine de pois ;
par rapport aux protéines totales dans la fraction protéique, dans laquelle la somme desdites protéines est de préférence égale à 100 % en poids de la fraction protéique.

3. Composition nutritionnelle liquide pour utilisation selon la revendication 1 ou 2,
dans laquelle la fraction protéique fournit un motif d'acides aminés équilibré, avec des nombres en grammes pour 100 grammes de la fraction protéique :
| | |
|---|---|
| Histidine | de 2,0 à 2,6 |
| Isoleucine | de 5,2 à 6,4 |
| Leucine | de 9,0 à 11,0 |
| Lysine | de 7,5 à 9,0 |
| Méthionine | de 1,7 à 2,3 |
| Cystéine | de 1,1 à 1,7 |
| Thréonine | de 4,9 à 6,2 |
| Tryptophane | de 1,2 à 1,6 |
| Valine | de 5,5 à 6,9 |
| Phénylalanine | de 4,2 à 5,2 |
| Tyrosine | de 3,7 à 4,7 |

4. Composition nutritionnelle liquide pour utilisation selon l'une quelconque des revendications précédentes, pour utilisation dans la prévention et/ou le traitement du déclin musculaire, ou pour utilisation dans l'amélioration de la fonction musculaire.

5. Composition nutritionnelle liquide pour utilisation selon l'une quelconque des revendications précédentes, dans laquelle les protéines sont sous forme hydrolysée ou intacte, de préférence sous forme intacte.

6. Composition nutritionnelle liquide pour utilisation selon la revendication 5, dans laquelle il n'y a pas d'acides aminés, de peptides ou d'hydrolysats libres dans la composition.

7. Composition nutritionnelle liquide pour utilisation selon l'une quelconque des revendications précédentes, dans laquelle le sujet humain est un sujet humain âgé d'au moins 60 ans.

8. Composition nutritionnelle liquide pour utilisation selon l'une quelconque des revendications 4 à 7, dans laquelle l'amélioration de la fonction musculaire implique une amélioration de la synthèse des protéines musculaires.

9. Composition nutritionnelle liquide pour utilisation selon l'une quelconque des revendications précédentes, dans laquelle le sujet humain est un sujet humain âgé qui souffre ou risque de développer une sarcopénie ou une fragilité.

10. Composition nutritionnelle liquide pour utilisation selon l'une quelconque des revendications précédentes, dans laquelle la quantité quotidienne de protéines administrée au sujet humain est comprise entre 1,0 et 1,8 g/kg de poids corporel.

11. Composition nutritionnelle liquide pour utilisation selon l'une quelconque des revendications précédentes, dans laquelle la composition est adaptée à une alimentation par sonde.

12. Composition nutritionnelle liquide pour utilisation selon l'une quelconque des revendications 1 à 9, dans laquelle la quantité de protéines administrée au sujet humain est comprise entre 10 et 30 g par portion, de préférence entre 15 et 25 g par portion.
